Europäisches Patentamt

European Patent Office

Office européen des brevets

(19)

(11) Publication number: **0 265 099 B1**

## (12) EUROPEAN PATENT SPECIFICATION

(45) Date of publication of patent specification: **22.01.92**

(51) Int. Cl.⁵: **A23J 3/00**, A23L 1/305, A61K 37/02

(21) Application number: **87308694.6**

(22) Date of filing: **01.10.87**

(54) **Nutrient composition.**

(30) Priority: **13.10.86 JP 242539/86**

(43) Date of publication of application:
**27.04.88 Bulletin 88/17**

(45) Publication of the grant of the patent:
**22.01.92 Bulletin 92/04**

(84) Designated Contracting States:
**DE FR GB**

(56) References cited:
**EP-A- 0 106 309**

**OUAL PLANT PLANT FOODS HUM NUTR, vol. 32, 1983, pages 411-423, Martinus Nijhoff/Dr W. Junk Publishers, The Hague, NL; J. ADLER-NISSEN et al.: "Improvement of the functionality of vegetable proteins by controlled enzymatic hydrolysis"**

**STARCH, vol. 33, no. 1, 1981, pages 18-22, Verlag Chemie, GmbH, Weinheim, DE; E. BERGHOFER et al.: "Enzymatische Hydrolyse von hitzekoaguliertem Kartoffelprotein"**

(73) Proprietor: **AJINOMOTO CO., INC.**
**5-8, Kyobashi 1-chome, Chuo-ku**
**Tokyo 104(JP)**

(72) Inventor: **Sohnaka, Ichiro**
**No. 1-1 Suzuki-cho Kawasaki-ku**
**Kawasaki-shi Kanagawa-ken(JP)**
Inventor: **Kobayashi, Tetuo**
**No. 1-1 Suzuki-cho Kawasaki-ku**
**Kawasaki-shi Kanagawa-ken(JP)**
Inventor: **Takami, Tohru**
**No. 1-1 Suzuki-cho Kawasaki-ku**
**Kawasaki-shi Kanagawa-ken(JP)**
Inventor: **Sasaki, Michiro**
**No. 1-1 Suzuki-cho Kawasaki-ku**
**Kawasaki-shi Kanagawa-ken(JP)**
Inventor: **Sumikawa, Michito**
**No. 1-1 Suzuki-cho Kawasaki-ku**
**Kawasaki-shi Kanagawa-ken(JP)**
Inventor: **Yasuda, Naohiko**
**No. 1-1 Suzuki-cho Kawasaki-ku**
**Kawasaki-shi Kanagawa-ken(JP)**
Inventor: **Ozaki, Hachiro**
**No. 1-1 Suzuki-cho Kawasaki-ku**
**Kawasaki-shi Kanagawa-ken(JP)**

Inventor: **Kubota, Koji**
**No. 1-1 Suzuki-cho Kawasaki-ku**
**Kawasaki-shi Kanagawa-ken(JP)**


(74) Representative: **Bond, Bentley George et al**
**HASELTINE LAKE & CO. Hazlitt House 28**
**Southampton Buildings Chancery Lane**
**London, WC2A 1AT(GB)**

**Description**

The present invention relates to a novel nutrient composition for enteral or oral application to be used for mammals, and more specifically relates to a nutrient composition containing an enzymatic hydrolyzate of potato protein which contains as a main component a peptide composition having an average amino acid residue number of from 3 to 5.

The body proteins of mammals are always synthesized and then destroyed repeatedly, and are thus recycled. Therefore, proteins, particularly the essential amino acids (namely lysine, leucine, isoleucine, tryptophan, methionine, valine, phenylalanine and threonine) are necessary every day. In particular, under conditions where body protein breakdown is accelerated, for example under the condition of malnutrition or surgical invasion, the proper supplementation of proteins containing these essential amino acids is important.

Recently, nutritional supplementation in the medical field, including the surgical field, has brought about drastic therapeutic effects. These clinical nutritional methods include three kinds of methods, i.e. parenteral nutritional methods, enteral nutritional methods and oral nutritional methods. Although crystalline amino acids are used as a nitrogen source of parenteral nutrition, proteins and enzymatic hydrolyzates thereof, as well as crystalline amino acids, are used as nitrogen source of enteral and oral nutrition. As for the enteral and oral nutrition methods, a component nutrient agent wherein amino acids considered to be final products of digestion are used as a nitrogen source has hitherto been considered to be more effective for patients, particularly those who are lowered in digestion and absorption, than a component nutrient agent wherein proteins requiring digestion are used as a nitrogen source. However, it has recently been found that amino acids raise the osmotic pressure in the digestive tract to induce diarrhea. There are amino acids having low solubility and/or stability. Many of the final products of digestion are oligopeptides, and a specific transport system for peptides exists in the small intestine. Since the antagonism observed in the absorption of amino acids is less than that of peptides, the peptides are absorbed in a more effective way than amino acids (siik, D.B.A., et al., Peptides and Free Amino Acids, In Rombeau, J.L., Caldwell, M.D. (eds) : Clinical Nutrition, 1, W.B. Saunders Company (1984).

Therefore, it is considered to be effective to use an enzymatic hydrolyzate of a protein containing oligopeptides as a main component, as a nitrogen source, of enteral and oral nutrition.

Potato protein is manufactured by subjecting the residue obtained after starch has been removed from potato in a starch purification, to separation, concentration and drying. However, since the resulting protein is low in purity and contains nitrogen compounds in a hardly digestible form, it is inferior to a purified protein such as casein or lactoalbumin in respect of digestibility.

Furthermore, potato protein is colored and hardly soluble in water, which causes problems in processing, and is poor in texture. Thus, potato protein is unsuitable as a protein material of enteral and oral nutrition agents and used only as a raw material in proteins for feedstuffs. As is understood from the foregoing, potato protein has many problems in respect of digestibility, absorbability, solubility and appearance, and therefore very little research in respect of its nutrient properties has been carried out.

In order to utilize potato protein (a vegetable protein) as a nitrogen source of enteral and oral nutrition, the development of techniques for enhancing the digestibility and absorbability and the nutritional value thereof, and at the same time improving the solubility, colour and texture therefore, is desired.

The enzymatic hydrolysis of potato protein to improve the solubility thereof is disclosed in Starch, 33-(1), 18-22 (1981).

According to the present invention, there is provided a nutrient composition comprising (1) a peptide composition having an average amino acid residue number of from 3 to 5, obtainable by enzymatic hydrolysis of potato protein, and (2) at least one nutrient selected from fats, polyols, glucose, oligosaccharides, minerals, trace elements and vitamins.

The present invention also provides a process for preparing a nutrient composition, which comprises:

(a) preparing a peptide composition by dispersing potato protein in water so as to make the concentration 2 to 20 weight/volume %, and by causing protease to act on the protein to decompose it so as to give peptides having an average amino acid residue number of from 3 to 5; and

(b) preparing a nutrient composition comprising (1) the peptide composition and (2) at least one nutrient selected from fats, polyols, glucose, oligosaccharides, minerals, trace elements and vitamins.

The nutrient composition of the present invention exhibits an excellent intestinal absorption efficiency and an extremely high nutritional value, which is different from the case of amino acid compositions of potato protein. It does not cause side effects such as diarrhea owing to high osmotic pressure, and has a high solubility and a high stability.

The potato protein used in the present invention can be easily prepared, for example, by subjecting the

3

residue obtained after starch has been removed from potato in a starch purification step, to separation, concentration and drying, and can also be cheaply purchased.

Reference will be made hereinbelow to the drawings in which:

Fig. 1 is a graph exhibiting the change of total amino acid concentration in the portal plasma, with time, when a peptide composition comprising the nutrient composition of the present invention, and a conventional preparation, are respectively duodenally administered to male SD rats;

Fig. 2 is a graph similarly exhibiting the total amino acid concentration in the portal plasma, 5 minutes after the administration;

Figs. 3 and 4 respectively exhibit the nitrogen balance on a daily basis in the case of gastrectomized or enterectomized rats (see Example 3 below); and

Fig. 5 gives amino acid compositions.

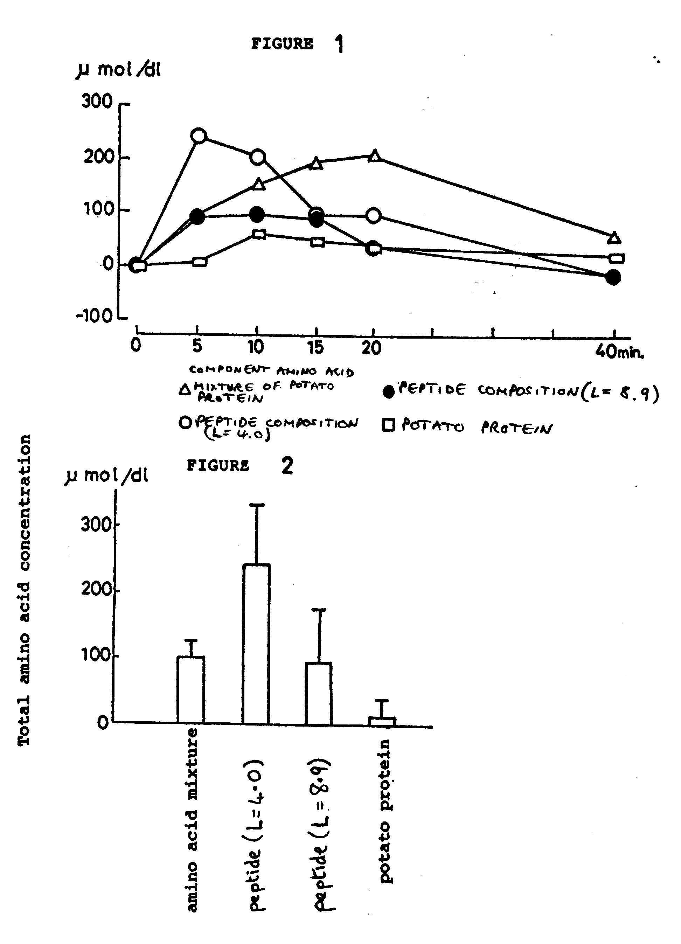

In Fig. 1, the various symbols have the following meanings:

△ : component amino acid mixture of potato protein;

○ : peptide composition (L = 4.0);

● : peptide composition (L = 8.9);

□ : potato protein.

The preparation of the peptide composition used in the present invention from potato protein is preferably carried out by dispersing potato protein as a raw material in water so as to make the concentration 2 to 20 weight/volume %, and making an enzyme act thereon. Proteases belonging to acid, neutral or alkaline protease derived from animals, plants, microorganisms, and the like can be used at the enzymes, and proteases originating from Bacillus subtilis are preferably used since they can restrain the formation of free amino acids and make the available amino acid residue number of the product 3 to 5.

Futhermore, the desired peptides can also be obtained by making a protease such as trypsin (which restrains the formation of free amino acids to a low level but gives a product having a large average amino acid residue number) act on potato protein, and successively making a protease such as subtilisin (which gives an average amino acid residue number of 3 to 5) act on the resulting product.

Conventional reaction conditions for enzymatic reactions can be used as the reaction conditions. Namely, the pH is usually 3 to 11, the temperature is usually 30 to 60°C and the reaction time is usually 1 to 48 hours. For example, when a neutral or alkaline protease originating from Bacillus subtilis is used, a pH of 5 to 9, a temperature of 40 to 50°C and a reaction time of 1 to 24 hours are preferred. The reaction can be discontinued according to a usual enzyme inactivating treatment, for example by heating the reaction mixture of 80°C or more for 5 minutes or more or by adding an acid or alkali thereto. In some cases, it is possible to stop the reaction by directly removing the enzyme by ultrafiltration without conducting the above inactivating treatment. Recovery of the desired peptides from the reaction mixture can easily be carried out by removing the insoluble matter by centrifugation or filtration, and subjecting the resulting solution to a usual drying method such as a freeze drying method or a spray drying method. Thereby, the desired product is obtained as a powder.

This hydrolyzate of potato protein ( a peptide composition) can be present in a nutrient composition which is to be orally or enteraly administered, usually in an amount of 1 to 25 weight %, preferably 10 to 20 weight %.

The enzymatic hydrolysis of potato proteins is preferably stopped at such a stage that the content of peptides having an average amino acid residue number of from 3 to 5 is 70% or more and the content of free amino acids is 15% or less, taking into account the influence which the extent of the hydrolysis has on the solubility and osmotic pressure.

Besides the free amino acids formed during the enzymatic hydrolysis, at least one of various amino acids, particularly crystals of essential amino acids and other amino acids such as lysine, leucine, isoleucine, tryptophan, methionine, valine, phenylalanine and threonine, may be added to the nutrient composition according to the catabolic condition such as malnutrition, surgical invasion or the like.

Nutrients such as fats, polyols, glucose, oligosaccharides, minerals, trace elements or vitamins, alone or in combination, are present in the nutrient composition.

As can be understood from the foregoing, the nutrient composition of the present invention, which contains a peptide composition which is obtained by the enzymatic hydrolysis of potato protein as a raw material, is excellent in intestinal absorption, has a high nutritional value and is excellent in physical properties such as solubility, and is ideal as a nutrient composition to be enterally or orally administered.

The present invention is illustrated by the following Examples.

Analytical methods were carried on the product of the present invention as follows:

(1) Average amino acid residue number (L)

4

The (L) value of the peptide part, excluding the influence of free amino acids and amino groups on the lysine side chains, was determined according to the following formulae:

$$l = \frac{\begin{bmatrix} \text{Mole number of amino} \\ \text{groups in the complete} \\ \text{acidic hydrolyzate of 1g} \\ \text{of enzymatic hydrolyzate} \\ \text{of potato protein} \end{bmatrix} - \begin{bmatrix} \text{Mole number of lysine} \\ \text{in 1g of enzymatic} \\ \text{hydrolyzate of potato} \\ \text{protein} \end{bmatrix}}{\begin{bmatrix} \text{Mole number of amino} \\ \text{groups in 1g of} \\ \text{enzymatic hydrolyzate} \\ \text{of potato protein} \end{bmatrix} - \begin{bmatrix} \text{Mole number of} \\ \text{lysine in 1g of} \\ \text{enzymatic hydrolyzate} \\ \text{of potato protein} \end{bmatrix}}$$

$$L = \frac{\frac{100-A}{100} - A}{l}$$

wherein:

$$A = \frac{\begin{bmatrix} \text{Total mole number of free amino acids} \\ \text{in 1g of enzymatic hydrolyzate of potato protein} \end{bmatrix}}{\begin{bmatrix} \text{Total mole number of amino acids in} \\ \text{the complete acidic hydrolyzate of 1g of} \\ \text{enzymatic hydrolyzate of potato protein} \end{bmatrix}}$$

The determination of the amino group was conducted according to the TNBS method, and the complete hydrolysis was conducted by hydrolyzing the product in 6 N HCl at 110° C for 24 hours.

(2) Free amino acids

This analysis was conducted using a HITACHI 835 type amino acid automatically analyzing apparatus.

Example 1 Nutritional value

The nutritional value of potato protein was examined using an experimental meal having the composition given in Table 1 below, and further containing, as a nitrogen source, potato protein, potato protein plus 0.25% methionine, casein, casein plus 0.25% methionine or whole egg protein.

The five experimental meals were respectively freely ingested by male SD rats 5 weeks old for 4 weeks. As can be seen from Table 2 below, giving the results obtained, it was confirmed from the weight increase and the PER (Protein Efficiency Ratio) that potato protein has a high nutritional value in no way inferior to casein, and is improved to have almost the same nutritional value as whole egg protein by supplementing methionine which is the first limiting amino acid.

Example 2 Intestinal absorption characteristics

Protease was made to act on potato protein to prepare two kinds of peptides respectively having an average amino acid residue number (L) of 4.0 and 8.9. The peptides were used for the examination of

5

intestinal absorption characteristics and enteral nutritional effects. In this respect, a component amino acid mixture of potato and solubilised potato protein were used as controls, respectively, in the same nitrogen amount as the peptide.

Aqueous 3% solutions of each of the two kinds of peptides, the component amino acids of potato protein, and the potato protein were duodenally administered under no anesthesia and no restraint to male SD rats (300 to 350g) to which a portal vein catheter and a duodenal catheter were fixed, and which had been made to fast for 24 hours. Blood was collected from the portal vein of each animal at regular intervals, the intestinal absorption characteristics were measured, and the results obtained are as given in Figures 1 and 2.

The absorption amount and the initial absorption rate were respectively determined from the change of total amino acid concentration in the portal plasma with time (Fig. 1) and the total amino acid concentration in the portal vein plasma 5 minutes after the administration. As for the absorption amount, the peptide composition having an average amino acid residue number of 4.0, and the component amino acid mixture of potato protein, respectively, exhibited a value higher than the peptide composition having an average amino acid residue number of 8.9 and the potato protein. As for the initial absorption rate, it was confirmed that the peptide composition having an average amino acid residue number of 4.0 was absorbed about twice as fast as the component amino acids of potato protein and the peptide composition having an average amino acid residue number of 8.9, and about 20 times as fast as the potato protein. That is to say in the case of intestinal administration, the protein and the high molecular peptides requiring a digesting stage became lower in absorptivity, and the amino acids became lower in initial absorption rate owing to absorption antagonism. On the other hand, the oligopeptides exhibited a higher absorptivity and a higher initial absorption rate because they were absorbed through a specific transport system thereto, and some of them are acted on by a peptidase on the epitheliocyte brush border membrane and then absorbed through an amino acid transport system.

Example 3 Enteral nutritional effect

An enteral diet (ED) having the composition given in Table 3 below and the same nitrogen content and the same energy as one another were prepared using the four above-mentioned kinds of samples, and used for research into the enteral nutritional effect thereof. A 33.4% ED solution was continuously infused for 7 days into the jejunum of male SD rats, 5 weeks old, which had been made to fast overnight, and the nitrogen efficiency ratio (NER) (Table 4 below), nitrogen balance (Table 5 below), and serum protein (Table 6 below) were determined. It was confirmed that the peptides having an average amino acid residue number of 4.0 and the component amino acid mixture of potato exhibited values higher than the peptides having an average amino acid residue number of 8.9 and the potato protein, in respect of weight gain (NER), nitrogen balance, serum total protein (TP), and serum albumin (ALB), and had a higher enteral nutritional effect.

EDs were prepared as described above using (a) the peptide composition having an average amino acid residue number of 4.0 and (b) the component amino acid mixture of potato, and compared in respect of enteral nutritional effect on digestive apparatus-invaded rats whose ability of digestion and absorption was surgically lowered.

The nitrogen balance on a daily basis when a 33.4% solution of each ED was infused into the jejunum of gastrectomized rats for 17 days in shown in Fig. 3. The peptides having an average amino acid residue number of 4.0 exhibited higher values in respect of nitrogen retention through the experimental period, and significantly higher value 5 days after the operation, compared with the component amino acid mixture of potato protein. The nitrogen balance on a daily basis when a 33.4% ED solution was infused into the duodenum of enterectomized rats for 7 days is shown in Fig. 4. The peptides having an average amino acid residue number of 4.0 exhibited higher values in respect of nitrogen retention through the experimental period, and significantly higher values 3 days and 7 days after the operation, compared with the component amino acid mixture of potato protein.

Furthermore, the cholesterol-lowering effect of soybean protein has recently been reported by many researchers, and the enzymatic hydrolyzate of potato protein of the present invention was found to have a similar cholesterol-lowering effect, which suggests the usefulness thereof as a diet for the treatment of hyperlipidemia.

It may be concluded from the above experimental results that a low molecular peptide composition having an average amino acid residue number of 3 to 5, obtained by enzymatically hydrolyzing potato protein, is very useful as a nitrogen source for enteral and oral nutrition.

Example 4 Colour

The enzymatic hydrolyzate of potato protein gives a clear solution free of turbidity, as shown in Table 7 below, because the colour originating from the potato protein is readily decoloured by an active carbon treatment.

Example 5 Solubility

The enzymatic hydrolyzate of potato protein has a solubility of about 10 times that of the component amino acid mixture of potato protein, as shown in table 8 below, and is thus superior thereto in respect of solubility.

Example 6 Osmotic pressure

Osmotic pressure, which is considered to be a cause of diarrhea, is lower in the case of the enzymatic hydrolyzate of potato protein than in the case of the component amino acid mixture of potato protein, as shown in Table 9 below.

It is thus seen from the above that the enzymatic hydrolyzate of potato protein is superior to potato protein in respect of transparency, and to the component amino acid mixture of potato in respect of solubility and osmotic pressure.

Preparation Example (hydrolysis of potato protein)

Firstly, 2kg of potato protein for feed, as commercially available, was dispersed in 40 litres of water, and the dispersion was adjusted to a pH 7.0 with 2N sodium hydroxide solution. Then, 26.7g of a neutral protease (ORIENTASE 90N, manufactured by Ueda Chemical Industry Co., Ltd.) was added thereto. The mixture was reacted at 50°C for 24 hours and heated at 80°C for 30 minutes to inactivate the enzyme and stop the reaction. The filtrate, after insoluble matter had been removed by filtration, was adjusted to a pH of 5.0 with 1 N HC1. Then, 172g of active carbon were added thereto, and the mixture was stirred at 30°C for 2 hours so as to decolor it. After removal of the active carbon by filtration, the filtrate was adjusted to a pH of 6 with 2N NaOH, subjected to filtration through an ultrafiltration film (PM-10, manufactured by Romicon Inc.), and dried with a spray drier to obtain 860g of powder. This preparation had an average amino acid residue number of 4.2 and contained 4.4% of free amino acid.

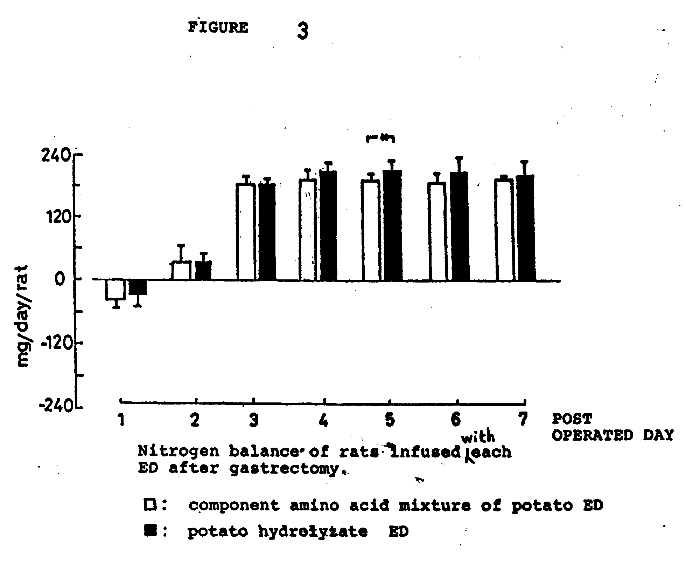

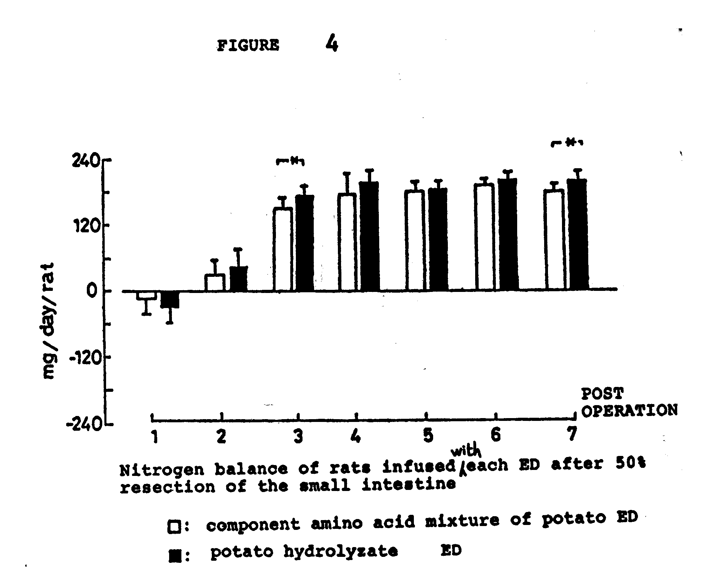

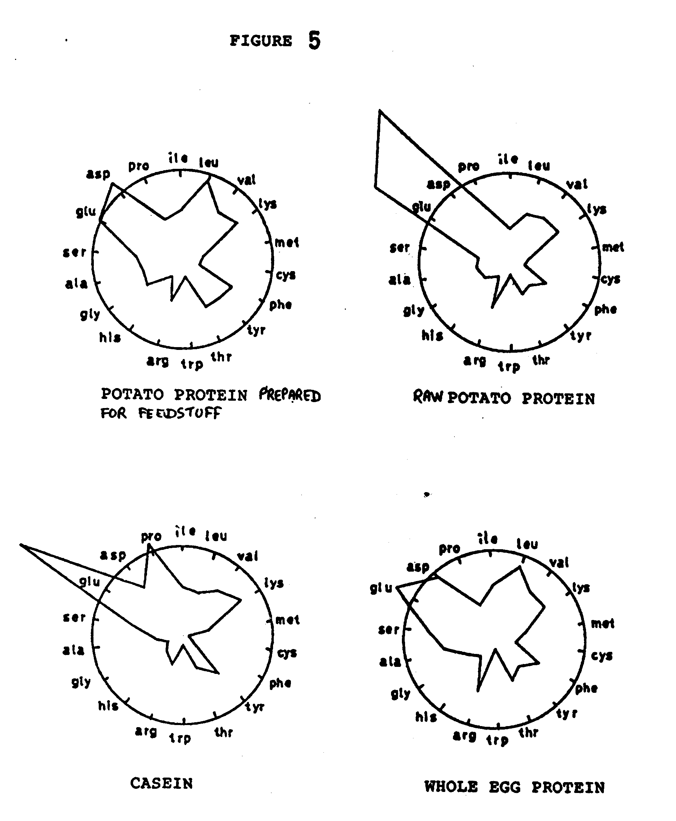

The respective amino acid compositions of the potato protein prepared for feedstuff and used in the present preparation method, of raw potato protein, of casein, and of whole egg protein are shown in Fig. 5.

Example 7 Diet for enteral nutrition

A diet for enteral nutrition was prepared according to the following method using the enzymatic hydrolyzate of potato protein having an average amino acid residue number of 4.2 as obtained in the Preparation Example.

636g of soybean oil, 21.0g of soybean phospholipid, 24.5g of polysorbate 80 were uniformly mixed to prepare a Mixture I.

16.2 g of retinol acetate granules, 242mg of thiamine hydrochloride, 320mg of sodium riboflavin phosphate, 334mg of pyridixone hydrochloride, 0.9mg of cyanocobalamin, 9.75g of ascorbic acid, 1.6mg of ergocalciferol, 20.6mg of tocopherol acetate granules, 1.49g of calcium pantothenate, 2.75g of nicotinamide, 49g of biotin, 55mg of folic acid, 22.4 g of choline bitartrate, and 11mg of phytonadion were ground and mixed to prepare a Mixture II.

19.4g of iron gluconate dihydrate, 10.3g of copper sulfate pentahydrate, 16.3g of manganese sulfate pentahydrate, 9.85g of zinc sulfate heptahydrate, 24.5mg of potassium iodide and 506.7g of magnesium sulfate were ground and mixed to prepare a Mixture III.

188g of potassium chloride, 770g of sodium citrate dihydrate, 1.031g of calcium glycerophosphate and 150g of potassium sorbate were ground and mixed to prepare a Mixture IV.

The whole amount of each of mixtures I, II, III and IV, and 19.86kg of the enzymatic hydrolyzate of potato protein, were mixed, with the addition of dextrin, to prepare 100kg of a transintestinal nutrient agent.

Example 8

3.5kg of soybean oil, 340g of soybean phospholipid, 50.0g of polysorbate 80, 2.0 g of L-ascorbic and stearic ester were uniformly mixed to prepare a Mixture V.

27.0g of retinol acetate granules, 404mg of thiamine hydrochloride, 534mg of sodium riboflavin phosphate, 556mg of pydridoxine hydrochloride, 1.5mg of cyanocobalamin, 35.8g of ascorbic acid, 10.7mg of ergocalciferol, 34.4g of tococopherol acetate granules, 2.48g of calcium pantothenate, 4.58g of nicotinamide, 81.7 mg of biotin, 91.7mg of folic acid, 105g of choline bitartrate and 1.83g of phytonadion powder were ground and mixed to prepare a Mixture VI.

55.0 g of iron gluconate dihydrate, 72g of copper sulfate pentahydrate (or 72g of manganese sulfate heptahydrate), 16.4g of zinc sulfate heptahydrate and 268.4g of magnesium sulfate heptahydrate were ground and mixed to obtain a Mixture VII.

962.1g of potassium chloride, 1.54kg of sodium citrate dihydrate, 2.23kg of calcium glycerophosphate, 40.8mg of potassium iodide and 150g of potassium sorbate were ground and mixed to prepare a Mixture VIII.

The whole amount of each of mixtures V, VI, VII and VIII, 13.90kg of the enzymatic hydrolyzate of potato protein and 273.4g of L-methionine were mixed, with the addition of dextrin, to prepare 100kg of a diet for enteral nutrition.

Table 1

| Composition | % |
|---|---|
| Protein (corresponding to 1.6 % N) Corn starch | 85.8 |
| Soybean oil | 5.0 |
| Mineral mixture | 4.0 |
| Vitamin mixture | 1.0 |
| Choline chloride | 0.2 |
| Cellulose | 4.0 |

Table 2

| | n | Initial weight | Final weight | Weight gain | Food intake (Nitrogen intake) | PER |
|---|---|---|---|---|---|---|
| | | g | g | g/7 days | g/7 days | |
| potato | 10 | 119.1±5.0 | 270.1±19.8[b] | 151.0±18.2[b] | 529.5±42.5 (8.47±0.68)[a] | 2.85±0.19 c |
| potato +Met | 9 | 117.1±4.8 | 305.3±23.0[a] | 187.6±20.5[a] | 555.7±48.3 (8.89±0.77)[a] | 3.37±0.12 b |
| casein | 10 | 117.4±5.1 | 251.0±22.3[b] | 133.6±19.8[b] | 477.9±46.7 (7.65±0.75)[b] | 2.78±0.18 c |
| casein +Met | 10 | 118.6±4.6 | 309.0±24.4[a] | 190.4±24.5[a] | 554.2±38.8 (8.87±0.62)[a] | 3.43±0.23 ab |
| whole egg protein | 10 | 117.6±4.9 | 317.9±21.7[a] | 200.3±19.0[a] | 561.5±42.2 (8.98±0.68)[a] | 3.56±0.10 a |

a , b , c , d : $p < 0.05$ ( a > b > c > d )

EP 0 265 099 B1

### Table 3

| Sample (in an amount corresponding to 2.5% nitrogen) Dextrin | 96.47 g |
|---|---|
| Sodium citrate (dihydrate) | 770 mg |
| Potassium chloride | 188 mg |
| Calcium glycerophophate | 1,031 mg |
| Iron gluconate (dihydrate) | 19.4 mg |
| Zinc sulfate (heptahydrate) | 9.85 mg |
| Manganese sulfate (pentahydrate) | 1.63 mg |
| Copper sulfate (pentahydrate) | 1.03 mg |
| Potassium iodide | 24.5 µg |
| Magnesium sulfate (heptahydrate) | 507 mg |
| Thiamine hydrochloride | 242 µg |
| Sodium riboflavin phosphate | 320 µg |
| Pyridoxine hydrochloride | 334 µg |
| Cyanocobalamin | 0.9 µg |
| Calcium pantothenate | 1.49 mg |
| Nicotinamide | 2.75 mg |
| Folic acid | 55 µg |
| Biotin | 49 µg |
| Choline bitartrate | 22.41 mg |
| Ascorbic acid | 9.75 mg |
| Retinol acetate granule | 16.2 mg |
| Tocopherol acetate granule | 20.63 mg |
| Ergocalciferol | 1.6 µg |
| Rhytonadion | 1.1 µg |
| Soybean oil | 635 mg |
| Potassium sorbate | 150 mg |
| Polysorbate 80 | 24.5 mg |
| Soybean phospholipid | 21.0 mg |

EP 0 265 099 B1

Table 4

| | n | Weight gain | Infused nitrogen amount | NER |
|---|---|---|---|---|
| | | $g$ / 7 days | $g$ / 7 days | |
| Component amino acid mixture of potato ED | 5 | $38.3 \pm 4.9^a$ | $2.44 \pm 0.13$ | $15.7 \pm 1.3^a$ |
| Potato hydrolyzate ( L=4.0 ) ED | 13 | $40.3 \pm 3.3^a$ | $2.42 \pm 0.10$ | $16.7 \pm 1.1^a$ |
| Potato hydrolyzate ( L=8.9 ) ED | 5 | $23.0 \pm 3.8^b$ | $2.48 \pm 0.05$ | $9.30 \pm 1.67^b$ |
| Potato protein ED | 7 | $21.1 \pm 2.7^b$ | $2.45 \pm 0.13$ | $8.60 \pm 0.91^b$ |

Table 5

| | n | Infused N | N in urine | N in feces | N balance | Digestivity [a] |
|---|---|---|---|---|---|---|
| | | mg/3days | mg/3days | mg/3days | mg/3days | % |
| Component amino acid of potato ED (mixture) | 5 | 1188.8±66.7 | 533.8±34.5[a] | 19.8±9.6[d] | 635.6±46.5[a] | 98.3±0.9[a] |
| Potato hydrolyzate (L=4.0)ED | 13 | 1218.5±50.7 | 518.8±46.9[a] | 58.7±20.5[c] | 645.7±49.6[a] | 95.2±1.6[b] |
| Potato hydrolyzate (L=8.9)ED | 5 | 1200.6±27.8 | 359.5±55.4[c] | 197.8±49.9[b] | 643.3±39.5[a] | 83.6±3.8[c] |
| Potato protein ED | 7 | 1237.0±39.8 | 458.9±43.2[b] | 319.1±37.9[a] | 459.0±48.2[b] | 74.4±3.7[d] |

EP 0 265 099 B1

Table 6

| | n | Total protein g/dℓ | Albumin g/dℓ |
|---|---|---|---|
| Component amino acid mixture of potato ED | 5 | $5.21 \pm 0.21^{a}$ | $2.38 \pm 0.12^{a}$ |
| Potato hydrolyzate ( L = 4.0 ) ED | 13 | $5.05 \pm 0.17^{a}$ | $2.22 \pm 0.17^{a}$ |
| Potato hydrolyzate ( L = 8.9 ) ED | 5 | $4.75 \pm 0.10^{b}$ | $1.79 \pm 0.17^{b}$ |
| Potato protein ED | 7 | $4.82 \pm 0.17^{b}$ | $1.89 \pm 0.13^{b}$ |

EP 0 265 099 B1

Table 7

|  | Permeability at 430nm |
|---|---|
| Component amino acid mixture of potato<br>Potato hydrolyzate (L = 4. 0)<br>Potato protin | 99. 0%<br>90. 2%<br>38. 2% |
| (5% aqueous solution) (pH 6. 0) |  |

Table 8

|  | Solubility in water |
|---|---|
|  | g |
| Component amino acid mixture of potato<br>Potato hydrolyzate | <6<br>about 100 |
| (Water 100ml, pH6. 5. 25 $^{\circ}$ C) |  |

Table 9

|  | 3% aqueous soluton | 5% aqueous solution |
|---|---|---|
|  | mOs m/ℓ | |
| Component amino scid mixture of potato<br>Potato hydrolyzate (L = 4. 0) | 190<br>81 | 387<br>160 |

## Claims

1. A nutrient composition comprising (1) a peptide composition having an average amino acid residue number of from 3 to 5, obtainable by enzymatic hydrolysis of potato protein, and (2) at least one nutrient selected from fats, polyols, glucose, oligosaccharides, minerals, trace elements and vitamins.

2. A nutrient composition according to claim 1, wherein the peptide composition contains peptides having an average amino acid residue number of from 3 to 5 in an amount of 70% or more, and free amino acids in an amount of 15% or less.

3. A nutrient composition according to claim 2, wherein the peptide composition is present in an amount of from 1 to 25 weight %.

4. A process for preparing a nutrient composition, which comprises:
   (a) preparing a peptide composition by dispersing potato protein in water so as to make the concentration 2 to 20 weight/volume %, and by causing protease to act on the protein to decompose it so as to give peptides having an average amino acid residue number of from 3 to 5; and
   (b) preparing a nutrient composition comprising (1) the peptide composition and (2) at least one nutrient selected from fats, polyols, glucose, oligosaccharides, minerals, trace elements and vitamins.

5. A process according to claim 4, wherein the protease is a neutral or alkaline protease originating from a microorganism of the genus Bacillus.

## Revendications

14

1. Composition nutritive comprenant (1) une composition peptidique ayant un nombre moyen de résidus aminoacide compris entre 3 et 5, qui peut être obtenue par hydrolyse enzymatique de protéine de pommme de terre et (2) au moins un nutriment sélectionné parmi les graisses, les polyols, le glucose, les oligosaccharides, les minéraux, les oligoéléments et les vitamines.

2. Composition nutritive selon la revendication 1, dans laquelle la composition peptidique contient des peptides ayant un nombre moyen de résidus aminoacide compris entre 3 et 5 en une quantité supérieure ou égale à 70 % et des aminoacides libres en une quantité inférieure ou égale à 15 %.

3. Composition nutritive selon la revendication 2, dans laquelle la composition peptidique est présente en une quantité comprise entre 1 et 25 % en poids.

4. Procédé de préparation d'une composition nutritive, comprenant :
   (a) la préparation d'une composition peptidique par dispersion de protéine de pomme de terre dans l'eau de façon à obtenir une concentration de 2 à 20 % en poids/volume et par action d'une protéase sur la protéine pour la décomposer de façon à donner des peptides ayant un nombre moyen de résidus aminoacide compris entre 3 et 5 ; et
   (b) la préparation d'une composition nutritive comprenant (1) la composition peptidique et (2) au moins un nutriment sélectionné parmi les graisses, les polyols, le glucose, les oligosaccharides, les minéraux, les oligoéléments et les vitamines.

5. Procédé selon la revendication 4, dans lequel la protéase est une protéase neutre ou alcaline provenant d'un microorganisme du genre <u>Bacillus</u>.

**Patentansprüche**

1. Nährstoffzusammensetzung, enthaltend (1) eine Peptidzusammensetzung mit einer mittleren Aminosäurerestzahl von 3 bis 5, die durch enzymatische Hydrolyse von Kartoffelprotein erhältlich ist, und (2) mindestens einen Nährstoff, ausgewählt unter Fetten, Polyolen, Glucose, Oligosacchariden, Mineralstoffen, Spurenelementen und Vitaminen.

2. Nährstoffzusammensetzung nach Anspruch 1, wobei die Peptidzusammensetzung Peptide mit einer mittleren Aminosäurerestzahl von 3 bis 5 in einem Anteil von 70 % oder mehr enthält und freie Aminosäuren in einem Anteil von 15 % oder weniger enthält.

3. Nährstoffzusammensetzung nach Anspruch 2, worin die Peptidzusammensetzung in einem Anteil von 1 bis 25 Gew.-% enthalten ist.

4. Verfahren zur Herstellung einer Nährstoffzusammensetzung, das umfaßt :
   (a) Herstellen einer Peptidzusammensetzung, indem Kartoffelprotein in Wasser dispergiert wird, so daß die Konzentration 2 bis 20 Gew./Vol.-% wird, und indem man Protease auf das Protein einwirken läßt, um es abzubauen, so daß Peptide mit einer mittleren Aminosäurerestzahl von 3 bis 5 erhalten werden; und
   (b) Herstellen einer Nährstoffzusammensetzung, enthaltend (1) die Peptidzusammensetzung und (2) mindestens einen Nährstoff, der unter Fetten, Polyolen, Glucose, Oligosacchariden, Mineralstoffen, Spurenelementen und Vitaminen ausgewählt ist.

5. Verfahren nach Anspruch 4, wobei die Protease eine neutrale oder alkalische Protease ist, die von einem Mikro-organismus der Gattung Bacillus stammt.

FIGURE 1

μ mol /dl

COMPONENT AMINO ACID
△ MIXTURE OF POTATO PROTEIN          ● PEPTIDE COMPOSITION (L= 8.9)

○ PEPTIDE COMPOSITION (L= 4.0)          □ POTATO PROTEIN

FIGURE 2

μ mol /dl

Total amino acid concentration

amino acid mixture
peptide (L=4.0)
peptide (L=8.9)
potato protein

16

FIGURE 3

Nitrogen balance of rats infused with each ED after gastrectomy.

□ : component amino acid mixture of potato ED

■ : potato hydrolyzate ED

17

FIGURE 4

Nitrogen balance of rats infused with each ED after 50% resection of the small intestine

☐: component amino acid mixture of potato ED

■: potato hydrolyzate  ED

FIGURE 5

POTATO PROTEIN PREPARED
FOR FEEDSTUFF

RAW POTATO PROTEIN

CASEIN

WHOLE EGG PROTEIN